# EUROPEAN PATENT APPLICATION

(11) **EP 3 195 864 A1**
(43) Date of publication of application: **26.07.2017**
(21) Application number: 15842123.0
(22) Date of filing: 17.09.2015
(51) Int. Cl.: A61K 31/407, A61K 31/46, A61K 31/435, A61K 31/137, A61K 31/472, A61K 31/235, A61K 31/24, A61K 31/133, A61K 31/192, A61K 9/14, A61K 9/16, A61K 9/54, A61P 29/00, A61P 1/06, A61P 13/06

(54) **PHARMACEUTICAL COMPOSITION CONTAINING AN ANALGESIC AGENT AND AN ANTISPASMOSDIC AGENT**

(30) Priority: 19.09.2014 BR 102014023319
(71) Applicant: Diffucap Chemobras Quimica E Farmaceutica Ltda., Quintino Bocaiuva, CEP-021380-010, RJ (BR)
(72) Inventor: ABRAMOWICZ, Jaime, 20550-170 - Rio de Janeiro - RJ (BR); SOUZA, Fernando Rafael de, 20973-070 - Rio de Janeiro - RJ (BR); MARTINS, Carlos Antonio, 20765-280 - Rio de Janeiro - RJ (BR)
(74) Representative: Camolese, Marco
(86) International application number: PCT/BR2015/000146
(87) International publication number: WO 2016/041036

(57) **Abstract**

"PHARMACEUTICAL COMBINATION CONTAINING AN ANALGESIC AGENT AND AN ANTISPASMODIC AGENT", whose analgesic agent is the Ketorolac Tromethamine, which is physically isolated from the antispasmodic agent and whose antispasmodic active ingredient, in combination with the analgesic agent Ketorolac Tromethamine, is Hyoscine, Pargeverine, Tolterodine, Mebeverine or Papaverine. Both the analgesic agent Ketorolac Tromethamine and the antispasmodic agent are combined in separate and immediate release form. The analgesic agent Ketorolac Tromethamine can also be found in the present combination in separate and immediate release form, while the combined antispasmodic agent is presented in equally separate, but programmed release form. The active ingredient Ketorolac Tromethamine is in a ratio between 2.5% and 20% by weight and the antispasmodic active ingredient is in a ratio between 2.5% and 20% by weight.

## Description

### FIELD OF APPLICATION

This application refers to a novel pharmaceutical combination comprising an analgesic, Ketorolac Tromethamine (carboxylic acid-5-Benzoyl-2,3-dihydro-1H-pyrrolisine-1, 2-amino-2-hydroxymethyl-1,3-propanediol) and a Hyoscine-like antispasmodic, or others like Pargeverine, Tolterodine, Mebeverine and Papaverine.

1 This application has as a characteristic, presented in a preferred type of embodiment, granules of each separate active ingredient in order to avoid contact incompatibility between the analgesic agent and the spasmodic agents, which form the present innovative patent application, the combination of which is described and claimed in the present patent.

Ketorolac Tromethamine is a non-steroidal anti-inflammatory with analgesic, anti-inflammatory and antipyretic action, whose mechanism of action is related to its ability to inhibit prostaglandin synthesis and a peripheral analgesic effect.

As an example, Hyoscine is an alkaloid synthesized by certain plants, which produces an anticholinergic effect, exerting an antispasmodic action on the smooth muscle of the gastrointestinal, bile and genitourinary tract.

While Pargeverine and Tolterodine are antispasmodic agents that competitively inhibit the activity of muscarinic receptors located mainly in the smooth muscle of some organs and glands.

Regarding Mebeverine and Papaverine, they have direct action on the smooth muscle of the gastrointestinal tract and act to relieve spasm with the consequent effect of smooth muscle relaxation.

### HISTORY

Ketorolac is known since the year 1978, from:
Belgian N° 856,681 and US N° 4,089,969 patents, which are mentioned as prior art of the present inventive activity in the scope of pharmacology.

Both patents belong to Syntex and define the Ketorolac in its preparation and its anti-inflammatory and analgesic action. Ketorolac is an inhibitor of the synthesis of prostaglandins with a peripheral analgesic effect from which no effect on opioid receptors has been demonstrated.

It is rapidly absorbed once oral and intramuscular administration occurs with a peak of plasma concentration between 1 and 2 hours and its average life varies between 4 and 8 hours. 99% of Ketorolac binds to plasma proteins and is administered every 6 hours; the plasma concentration level is reached at 24 hours, which may require a load dosage so as to shorten the period to achieve an important analgesic effect. The main route of elimination of Ketorolac and its metabolites (Para-hydroxylated and conjugates) is the urinary (92%), excreting the remainder through feces. The daily usual dosage varies from 40 mg to 80 mg, administering in several daily intakes and being the maximum dosage of 90 mg/day.

The chemical formula of Hyoscine (C₁₇H₂₁NO₄) differs from atropine only because it has an oxygen bond between carbon atoms 6 and 7, which gives it the possibility of penetrating the blood-brain barrier, even if the derived N-Butyl bromide used in this application does not cross it.

Combinations of antispasmodics with analgesics are known in the market, for the treatment of gastrointestinal spasmodic, bile ducts and genitourinary tract pains. These preparations combine the action of an antispasmodic with various analgesics such as *Paracetamol, Dipyrone* and *Ibuprofen,* there being no antecedent with respect to a combination of an antispasmodic with Ketorolac Tromethamine as an analgesic agent.

It has been shown that there is an important interaction between analgesics and antispasmodics in said preparations, which causes a high degree of neutralization of the effects of the active ingredients between them, ranging from 40% to 90%, causing a consequent decrease of their plasma concentrations.

Based on existing bibliography, the most important interaction between Ketorolac Tromethamine and an antispasmodic is verified in the combination with Hyoscine, for which reason the present application was designed in the preferred form of embodiment, in different granules for each separate active ingredient, avoiding contact and interaction thereof in the preparation.

### MAIN OBJECT

For the specified purposes, the present application refers to a novel pharmaceutical combination comprising programmed release granules in its formation, containing the analgesic Ketorolac Tromethamine (carboxylic acid-5-Benzoyl-2,3-dihydro-1H-prrrolisine-1, 2-amino-2-hydroxymethyl-1,3-propanediol) and an antispasmodic agent such as the Hyoscine, the Pargeverine, the Tolterodine, the Mebeverine and the Papaverine.

The composition of this new pharmaceutical combination was designed with the aim to avoid the interaction of the active ingredients in the preparation, as well as *in vivo,* once the preparation is ingested.

This is achieved by virtue of the type of microgranules to contain each active ingredient. Thus, the microgranules containing the analgesic agent Ketorolac Tromethamine are prepared to behave as immediate action, in order to rapidly achieve high plasma concentrations of the analgesic, while the microgranules containing the antispasmodic agent (Hyoscine, Pargeverine, Tolterodine, Mebeverine, Papaverine, among others) are prepared to behave as programmed action, achieving its gradual release in the organism, in different times and scopes of the analgesic.

### DESCRIPTION

In the following, it will be demonstrated, by way of no-limiting illustration, how the present application can be put into practice.

The granules containing the active ingredient Ketorolac Tromethamine are designed to be immediate release, being elaborated with cores composed of sugar and starch, which are incorporated to the micronized active ingredient, through the binder polymers.

The granules containing the antispasmodic active ingredient (Hyoscine, Pargeverine, Tolterodine, Mebeverine, Papaverine, among others), are designed to be immediate release, to which it is added to the microgranules, coating polymers in such a manner, to achieve the appropriate release profile.

Said granules present diameters between 0.2 mm and 1.8 mm, preferably, between 0.4 mm and 1.5 mm and add to these dyes o differentiate each containing the active ingredient.

The solvents used for the preparation of the substantially spherical granules can be acetone, isopropyl alcohol, ethyl alcohol, chloroform, methylene chloride, water or a mixture thereof.

As binder polymers, polyvinylpyrrolidones, polyethylene glicols, methylcellulose, sucrose, gelatin, starch and mixtures thereof are preferably used. As coating polymers or film formers, different types of methylcelluloses, hydroxypropylmethylcelluloses, hydroxypropylmethylcellulose phtalates, acrylic polymers (Eudragit L, S, RL, RS or combinations thereof), shellac and ethylcelluloses, combined in different ratios in acetone, alcoholic, aqueous solutions or mixtures thereof can be used. These solutions can have incorporated plasticizers of type of diethyl phthalate, dibutyl phthalate, polyethylene glycol, triethylcitrate, triacetin, triglycerides of fatty acids or others. As lubricant, talc is used.

Formula of the granules of *Ketorolac Tromethamine.*

| PRODUCT | FORMULA | |
|---|---|---|
| ***KETOROLAC TROMETHAMINE*** | **5** - **30** | **%** |
| POVIDONE (PVP K 30) | 1 - 5 | % |
| WHITE TALC | 5 - 30 | % |
| Tartrazine yellow DYE | 0.03 - 0.06 | % |
| CORES N° 1 | 88.97 - 34.94 | % |
| | 100.00 | % |

Formula of the granules of antispasmodic.

| PRODUCT | FORMULA | |
|---|---|---|
| ***ANTISPASMODIC*** | **5** - **30** | **%** |
| POVIDONE (PVP K 30) | 1 - 5 | % |
| WHITE TALC | 5 - 30 | % |
| Green DYE | 0.03 - 0.06 | % |
| CORES N° 1 | 88.97 - 34.94 | % |
| | 100.00 | % |

Composition as preferred mode of carrying out the contents of each capsule with separate microgranules, according to the active ingredient of immediate release both the analgesic and the antispasmodic.

| PRODUTO FINAL CÁPSULA | FORMULA | |
|---|---|---|
| ***CETOROLACO DE TROMETAMINA*** | **2**.**5** - **20** | **%** |
| ***ANTIESPASMÓDICO*** | **2**.**5** - **20** | **%** |
| POVIDONA (PVP K 30) | 1 - 5 | % |
| TALCO BRANCO | 5 - 30 | % |
| CORANTES (A+V) | 0.03 - 0.06 | % |
| NÚCLEOS | 88.97 - 34.94 | % |
| | 100.00 | % |

Composition as preferred mode of carrying out the contents of each capsule with separate microgranules, according to the active ingredient of programmed release for the antispasmodic case.

| FINAL PRODUCT CAPSULE | FORMULA | |
|---|---|---|
| ***KETOROLAC TROMETHAMINE*** | **2**.**5** - **20** | **%** |
| ***ANTISPASMODIC*** | **2.5 - 20** | **%** |
| POVIDONE (PVP K 30) | 1 - 5 | % |
| WHITE TALC | 5 - 30 | % |
| DYES (A+V) | 0.03 - 0.06 | % |
| CORES | 87.97 - 29.94 | % |
| COATING POLYMERS | 1 - 5 | % |
| | 100.00 | % |

There is no doubt that when the present application is put into practice, modifications may be made to certain construction details and form without departing from the fundamental principles which are clearly demonstrated in the following claims:

## Claims

1. "PHARMACEUTICAL COMBINATION CONTAINING AN ANALGESIC AGENT AND AN ANTISPASMODIC AGENT", wherein the analgesic agent contains Ketorolac Tromethamine and is present in said composition in a ratio of between 5% and 20% by weight and the antispasmodic active ingredient is in a ratio of between 5% and 20% by weight of said composition.

2. "PHARMACEUTICAL COMBINATION CONTAINING AN ANALGESIC AGENT AND AN ANTISPASMODIC AGENT", according to claim 1, wherein the analgesic agent Ketorolac Tromethamine is physically isolated from the antispasmodic agent.

3. "PHARMACEUTICAL COMBINATION CONTAINING AN ANALGESIC AGENT AND AN ANTISPASMODIC AGENT", according to claim 1, wherein the antispasmodic active ingredient in combination with the analgesic agent Ketorolac Tromethamine is Hyoscine.

4. "PHARMACEUTICAL COMBINATION CONTAINING AN ANALGESIC AGENT AND AN ANTISPASMODIC AGENT", according to claim 1, wherein the antispasmodic active ingredient in combination with the analgesic agent Ketorolac Tromethamine is Pargeverine.

5. "PHARMACEUTICAL COMBINATION CONTAINING AN ANALGESIC AGENT AND AN ANTISPASMODIC AGENT", according to claim 1, wherein the antispasmodic active ingredient in combination with the analgesic agent Ketorolac Tromethamine is Tolterodine.

6. "PHARMACEUTICAL COMBINATION CONTAINING AN ANALGESIC AGENT AND AN ANTISPASMODIC AGENT", according to claim 1, wherein the antispasmodic active ingredient in combination with the analgesic agent Ketorolac Tromethamine is Mebeverine.

7. "PHARMACEUTICAL COMBINATION CONTAINING AN ANALGESIC AGENT AND AN ANTISPASMODIC AGENT", according to claim 1, wherein the antispasmodic active ingredient in combination with the analgesic agent Ketorolac Tromethamine is Papaverine.

8. "PHARMACEUTICAL COMBINATION CONTAINING AN ANALGESIC AGENT AND AN ANTISPASMODIC AGENT", according to claim 1, wherein both the analgesic agent Ketorolac Tromethamine and the antispasmodic agent are combined in separate and immediate release form.

9. "PHARMACEUTICAL COMBINATION CONTAINING AN ANALGESIC AGENT AND AN ANTISPASMODIC AGENT", according to claim 1, wherein the analgesic agent Ketorolac Tromethamine is the present combination in separate and immediate release form, while the combined antispasmodic agent is presented in an equally separate, but programmed release form.

10. "PHARMACEUTICAL COMBINATION CONTAINING AN ANALGESIC AGENT AND AN ANTISPASMODIC AGENT", according to claim 1, wherein, for presentation in the form of granules containing the active ingredients, these have diameters between 0.2 mm and 1.8 mm, preferably between 0.4 mm and 1.5 mm.

11. "PHARMACEUTICAL COMBINATION CONTAINING AN ANALGESIC AGENT AND AN ANTISPASMODIC AGENT", according to claim 1, wherein the used binder polymer is polyvinylpyrrolidone (PVP K30).

12. "PHARMACEUTICAL COMBINATION CONTAINING AN ANALGESIC AGENT AND AN ANTISPASMODIC AGENT", according to claim 1, wherein the used binder polymer is polyethylene glycol.

13. "PHARMACEUTICAL COMBINATION CONTAINING AN ANALGESIC AGENT AND AN ANTISPASMODIC AGENT", according to claim 1, wherein the used binder polymer is methylcellulose.

14. "PHARMACEUTICAL COMBINATION CONTAINING AN ANALGESIC AGENT AND AN ANTISPASMODIC AGENT", according to claim 1, wherein the used binder polymer is amide and mixtures thereof.

15. "COMBINAÇÃO FARMACÊUTICA CONTENDO UM ANALGESIC AGENT E UM ANTISPASMODIC AGENT", according to claim 1, wherein the coating polymer for the programmed release of antispasmodic is ethyl cellulose in different proportions in acetone, alcoholic, aqueous solutions or mixtures thereof.

16. "PHARMACEUTICAL COMBINATION CONTAINING AN ANALGESIC AGENT AND AN ANTISPASMODIC AGENT", according to claim 1, wherein the coating polymer for the programmed release of antispasmodic is hydroxypropylmethylcellulose in different proportions in acetone, alcoholic, aqueous solutions or mixtures thereof.

17. "PHARMACEUTICAL COMBINATION CONTAINING AN ANALGESIC AGENT AND AN ANTISPASMODIC AGENT", according to claim 1, wherein the coating polymers for the programmed release of antispasmodic are acrylic polymers (Eudragit L, S, RL, RS or combinations thereof) in different proportions in acetone, alcoholic, aqueous solutions or mixtures thereof.

18. "PHARMACEUTICAL COMBINATION CONTAINING AN ANALGESIC AGENT AND AN ANTISPASMODIC AGENT", according to claim 1, wherein the present combination has incorporated plasticizers of the type of diethyl phthalates, dibutyl phthalates, polyethylene glicols, triethylcitrates, triacetin and triglycerides of fatty acids.
